# EUROPEAN PATENT APPLICATION

(11) **EP 3 689 336 A1**
(43) Date of publication of application: **05.08.2020**
(21) Application number: 18856283.9
(22) Date of filing: 07.09.2018
(51) Int. Cl.: A61K 9/127, A61K 39/00, A61K 39/44, A61K 39/395, A61K 47/00, A61P 35/00

(54) **MULTICELLULAR TARGETING LIPOSOME**

(30) Priority: 14.09.2017 CN 201710828962
(71) Applicant: Shanghai Jiao Tong University, Shanghai 200240 (CN)
(72) Inventor: XU, Yuhong, Shanghai 200240 (CN); XIE, Fang, Shanghai 200240 (CN)
(74) Representative: Baudler, Ron
(86) International application number: PCT/CN2018/104533
(87) International publication number: WO 2019/052401

(57) **Abstract**

The present invention falls within the field of the use of liposome preparations in the preparation of tumor drugs, and in particular relates to the construction and use of a multicellular targeting liposome. A single liposome comprises multiple antibodies against different cells and a liposome with a stable structure and long circulation characteristics in vivo is constituted by molecular self-assembly. The multicellular targeting liposome can sequentially or simultaneously bind to multiple different cells by means of the action of antibodies to promote the identification of, communication between, the cytotoxicity of, the pro-apoptosis or clearance of different cells, etc.

SEQUENCE LISTING

<110>  SHANGHAI JIAO TONG UNIVERSITY

<120>  MULTICELLULAR TARGETING LIPOSOME

<130>  200023

<160>  16

<170>  PatentIn version 3.5

<210>  1
<211>  107
<212>  PRT
<213>  Artificial Sequence

<220>
<223>  The amino acid sequence of the light chain variable region of the
       CD19 antibody

<400>  1

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Arg Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Lys Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Trp
                85                  90                  95

Thr Phe Ala Gly Gly Thr Lys Leu Glu Ile Lys
            100                 105

<210>  2
<211>  122
<212>  PRT
<213>  Artificial Sequence

<220>
<223>  The amino acid sequence of the heavy chain variable region of the
       CD19 antibody

<400>  2

Glu Val Gln Leu Gln Gln Ser Gly Pro Glu Leu Val Lys Pro Gly Ala

1               5                   10                  15

Ser Met Lys Ile Ser Cys Lys Ala Ser Gly Tyr Ser Phe Thr Gly Tyr
            20                  25                  30

Thr Met Asn Trp Val Lys Gln Ser His Gly Lys Asn Leu Glu Trp Met
        35                  40                  45

Gly Leu Ile Asn Pro Tyr Lys Gly Val Ser Thr Tyr Asn Gln Lys Phe
    50                  55                  60

Lys Asp Lys Ala Thr Leu Thr Val Asp Lys Ser Ser Ser Thr Ala Tyr
65                  70                  75                  80

Met Glu Leu Leu Ser Leu Thr Ser Glu Asp Ser Ala Val Tyr Tyr Cys
                85                  90                  95

Ala Arg Ser Gly Tyr Tyr Gly Asp Ser Asp Trp Tyr Phe Asp Val Trp
            100                 105                 110

Gly Gln Gly Thr Thr Leu Thr Val Phe Ser
        115                 120

<210>  3
<211>  236
<212>  PRT
<213>  Artificial Sequence

<220>
<223>  The LC sequence of the Her2 antibody

<400>  3

Met Asp Met Arg Val Pro Ala Gln Leu Leu Gly Leu Leu Leu Leu Trp
1               5                   10                  15

Leu Arg Gly Ala Arg Cys Asp Ile Gln Met Thr Gln Ser Pro Ser Ser
            20                  25                  30

Leu Ser Ala Ser Val Gly Asp Arg Val Thr Ile Thr Cys Arg Ala Ser
        35                  40                  45

Gln Asp Val Asn Thr Ala Val Ala Trp Tyr Gln Gln Lys Pro Gly Lys
    50                  55                  60

Ala Pro Lys Leu Leu Ile Tyr Ser Ala Ser Phe Leu Tyr Ser Gly Val
65                  70                  75                  80

Pro Ser Arg Phe Ser Gly Ser Arg Ser Gly Thr Asp Phe Thr Leu Thr
                85                  90                  95

Ile Ser Ser Leu Gln Pro Glu Asp Phe Ala Thr Tyr Tyr Cys Gln Gln
                100                 105                 110

His Tyr Thr Thr Pro Pro Thr Phe Gly Gln Gly Thr Lys Val Glu Ile
        115                 120                 125

Lys Arg Thr Val Ala Ala Pro Ser Val Phe Ile Phe Pro Pro Ser Asp
    130                 135                 140

Glu Gln Leu Lys Ser Gly Thr Ala Ser Val Val Cys Leu Leu Asn Asn
145                 150                 155                 160

Phe Tyr Pro Arg Glu Ala Lys Val Gln Trp Lys Val Asp Asn Ala Leu
                165                 170                 175

Gln Ser Gly Asn Ser Gln Glu Ser Val Thr Glu Gln Asp Ser Lys Asp
                180                 185                 190

Ser Thr Tyr Ser Leu Ser Ser Thr Leu Thr Leu Ser Lys Ala Asp Tyr
        195                 200                 205

Glu Lys His Lys Val Tyr Ala Cys Glu Val Thr His Gln Gly Leu Ser
    210                 215                 220

Ser Pro Val Thr Lys Ser Phe Asn Arg Gly Glu Cys
225                 230                 235


<210>  4
<211>  237
<212>  PRT
<213>  Artificial Sequence

<220>
<223>  The HC sequence of the Her2 antibody

<400>  4

Met Glu Phe Gly Leu Ser Trp Val Phe Leu Val Ala Ile Leu Lys Gly
1               5                   10                  15

Val Gln Cys Glu Val Gln Leu Val Glu Ser Gly Gly Gly Leu Val Gln
            20                  25                  30

Pro Gly Gly Ser Leu Arg Leu Ser Cys Ala Ala Ser Gly Phe Asn Ile
        35                  40                  45

Lys Asp Thr Tyr Ile His Trp Val Arg Gln Ala Pro Gly Lys Gly Leu
    50                  55                  60

Glu Trp Val Ala Arg Ile Tyr Pro Thr Asn Gly Tyr Thr Arg Tyr Ala
65                  70                  75                  80

Asp Ser Val Lys Gly Arg Phe Thr Ile Ser Ala Asp Thr Ser Lys Asn
                85                  90                  95

Thr Ala Tyr Leu Gln Met Asn Ser Leu Arg Ala Glu Asp Thr Ala Val
            100                 105                 110

Tyr Tyr Cys Ser Arg Trp Gly Gly Asp Gly Phe Tyr Ala Met Asp Tyr
        115                 120                 125

Trp Gly Gln Gly Thr Leu Val Thr Val Ser Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val
225                 230                 235

<210>  5
<211>  119
<212>  PRT
<213>  Artificial Sequence

<220>
<223>  The VH sequence of the scFv of the EGFR antibody

<400>  5

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ala
        115

<210>  6
<211>  107
<212>  PRT
<213>  Artificial Sequence

<220>
<223>  The VL sequence of the EGFR antibody

<400>  6

Asp Ile Leu Leu Thr Gln Ser Pro Val Ile Leu Ser Val Ser Pro Gly
1               5                   10                  15

Glu Arg Val Ser Phe Ser Cys Arg Ala Ser Gln Ser Ile Gly Thr Asn
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Asn Asn Asn Trp Pro Thr
                85                  90                  95

Thr Phe Gly Ala Gly Thr Lys Leu Glu Leu Lys
            100                 105

<210>  7

<211>  121
<212>  PRT
<213>  Artificial Sequence

<220>
<223>  The VH sequence of the PSMA antibody

<400>  7

Gln Val Gln Leu Val Glu Ser Gly Gly Gly Leu Val Lys Pro Gly Glu
1               5                   10                  15

Ser Leu Arg Leu Ser Cys Ala Ala Ser Gly Phe Thr Phe Ser Asp Tyr
            20                  25                  30

Tyr Met Tyr Trp Val Arg Gln Ala Pro Gly Lys Gly Leu Glu Trp Val
        35                  40                  45

Ala Ile Ile Ser Asp Gly Gly Tyr Tyr Thr Tyr Tyr Ser Asp Ile Ile
    50                  55                  60

Lys Gly Arg Phe Thr Ile Ser Arg Asp Asn Ala Lys Asn Ser Leu Tyr
65                  70                  75                  80

Leu Gln Met Asn Ser Leu Lys Ala Glu Asp Thr Ala Val Tyr Tyr Cys
                85                  90                  95

Ala Arg Gly Phe Pro Leu Leu Arg His Gly Ala Met Asp Tyr Trp Gly
            100                 105                 110

Gln Gly Thr Leu Val Thr Val Ser Ser
        115                 120


<210>  8
<211>  107
<212>  PRT
<213>  Artificial Sequence

<220>
<223>  The VL sequence of the PSMA antibody

<400>  8

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Val Asp Thr Asn
            20                  25                  30

Val Ala Trp Tyr Gln Gln Lys Pro Gly Gln Ala Pro Lys Ser Leu Ile
        35                  40                  45

Tyr Ser Ala Ser Tyr Arg Tyr Ser Asp Val Pro Ser Arg Phe Ser Gly
    50                  55                  60

Ser Ala Ser Gly Thr Asp Phe Thr Leu Thr Ile Ser Ser Val Gln Ser
65                  70                  75                  80

Glu Asp Phe Ala Thr Tyr Tyr Cys Gln Gln Tyr Asp Ser Tyr Pro Tyr
                85                  90                  95

Thr Phe Gly Gly Gly Thr Lys Leu Glu Ile Lys
            100                 105

<210>  9
<211>  450
<212>  PRT
<213>  Artificial Sequence

<220>
<223>  The HC sequence of the PDL1 antibody

<400>  9

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ile Met Met Trp Val Arg Gln Ala Pro Gly Lys Gly Leu Glu Trp Val
        35                  40                  45

Ser Ser Ile Tyr Pro Ser Gly Gly Ile Thr Phe Tyr Ala Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ile Lys Leu Gly Thr Val Thr Thr Val Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser

145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  10
<211>  216
<212>  PRT
<213>  Artificial Sequence

<220>
<223>  The light chain sequence of the PDL1 antibody

<400>  10

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Arg Val Phe Gly Thr Gly Thr Lys Val Thr Val Leu Gly Gln
            100                 105                 110

Pro Lys Ala Asn Pro Thr Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Gly Ser Pro Val Lys

145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  11
<211>  119
<212>  PRT
<213>  Artificial Sequence

<220>
<223>  The VH sequence of the CD3 antibody

<400>  11

Asp Ile Lys Leu Gln Gln Ser Gly Ala Glu Leu Ala Arg Pro Gly Ala
1               5                   10                  15

Ser Val Lys Met Ser Cys Lys Thr Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser
        115

<210>  12
<211>  106

<212>  PRT
<213>  Artificial Sequence

<220>
<223>  The VL sequence of the CD3 antibody

<400>  12

Asp Ile Gln Leu Thr Gln Ser Pro Ala Ile Met Ser Ala Ser Pro Gly
1               5                   10                  15

Glu Lys Val Thr Met Thr Cys Arg Ala Ser Ser Ser Val Ser Tyr Met
            20                  25                  30

Asn Trp Tyr Gln Gln Lys Ser Gly Thr Ser Pro Lys Arg Trp Ile Tyr
        35                  40                  45

Asp Thr Ser Lys Val Ala Ser Gly Val Pro Tyr Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Trp Ser Ser Asn Pro Leu Thr
                85                  90                  95

Phe Gly Ala Gly Thr Lys Leu Glu Leu Lys
            100                 105


<210>  13
<211>  125
<212>  PRT
<213>  Artificial Sequence

<220>
<223>  The VH sequence of the CD3 antibody

<400>  13

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Asn Lys Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr

65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Ile Ser Tyr Trp
            100                 105                 110

Ala Tyr Trp Gly Gln Gly Thr Leu Val Thr Val Ser Ser
        115                 120                 125

<210>  14
<211>  110
<212>  PRT
<213>  Artificial Sequence

<220>
<223>  The VL sequence of the CD3 antibody

<400>  14

Gln Thr Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

Asn Tyr Pro Asn Trp Val Gln Gln Lys Pro Gly Gln Ala Pro Arg Gly
        35                  40                  45

Leu Ile Gly Gly Thr Lys Phe Leu Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Val Leu Trp Tyr Ser Asn
                85                  90                  95

Arg Trp Val Phe Gly Gly Gly Thr Lys Leu Thr Val Leu Ala
            100                 105                 110

<210>  15
<211>  113
<212>  PRT
<213>  Artificial Sequence

<220>
<223>  The VH sequence of the PD-1 antibody

<400>  15

<210>  16
<211>  111
<212>  PRT
<213>  Artificial Sequence

<220>
<223>  The VL sequence of the PD-1 antibody

<400>  16

Leu Glu Met Ala Glu Ile Val Leu Thr Gln Ser Pro Ala Thr Leu Ser
1               5                   10                  15

Leu Ser Pro Gly Glu Arg Ala Thr Leu Ser Cys Arg Ala Ser Gln Ser
            20                  25                  30

Val Ser Ser Tyr Leu Ala Trp Tyr Gln Gln Lys Pro Gly Gln Ala Pro
        35                  40                  45

Arg Leu Leu Ile Tyr Asp Ala Ser Asn Arg Ala Thr Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln Gln Ser Ser

                85                  90                  95

Asn Trp Pro Arg Thr Phe Gly Gln Gly Thr Lys Val Glu Ile Lys
            100                 105                 110

## Description

### Technical Field

The present disclosure belongs to the technical field of pharmaceutical preparations, liposome drug carriers and antibody targeting, and particularly relates to a multicellular targeting liposome pharmaceutical preparation that acts simultaneously with immune effector cells and target cells.

### Background

Liposome is a drug carrier with a bilayer vesicle-like structure based on phospholipid and/or cholesterol. Liposomes are artificially prepared bilayer vesicles formed by lipid molecules. Since the lipid molecule has a hydrophilic head and a hydrophobic tail, hydrophilic drugs can be contained in the internal aqueous phase, and hydrophobic drugs can be embedded in the lipid bilayer. In addition, liposomes have good histocompatibility, low toxicity and good biosecurity, therefore have attracted widespread attention and been applied in the field of drug delivery systems. In particular, the development of liposome targeting technology has endowed the lipid drug carrier with unique in vivo behavior, enabling it to enrich the target site and then interact with the target cell specifically. It is hoped that by optimizing the selection of targets and improving the structure and performance of targeting ligands, clinical efficacy of liposome drugs can be improved, including reducing drug system toxicity and increasing therapeutic index.

The research and development idea of targeting liposome drug delivery system aims at the receptors that are highly expressed or specifically expressed on the surface of tumor cells, to design various types of active targeting ligands and drug carriers. The active targeting drugs developed according to the above design idea have more optimized targeting capability at the lesion site than the free drugs delivered without targeting carriers, for example, the drug concentration at the target site is increased, the pharmacodynamics is improved, etc. However, this type of targeting drug delivery system only acts on a specific type of tumor cells, it cannot effectively clear the tumor, and may induce the problems such as minimal residual disease and multidrug resistance of the tumor.

In recent years, the understanding of tumor heterogeneity is improved, therefore the mechanism of the limitation of single-cell targeting has been elucidated. For example, the differences in tumor molecular markers between different patients, the differences in molecular markers between different tumor cells within the same tumor in the same patient, and the different types of cells and the interactions within the same tumor. Aiming at the characteristics of tumor heterogeneity, the researchers have expanded the design idea of targeting delivery. For example, the double-target liposome can enhance the binding ability between the carrier and the cell, and promote the uptake of drugs by the cell. Drug carriers targeting tumor-related cells can inhibit tumor growth through other mechanisms, such as acting on tumor new blood vessels, fibroblasts, and tumor stem cells.

Although such targeting delivery technology has improved therapeutic, the design idea still resides within the established framework of single-cell targeting. There are a wide range of dynamic interactions between different cells within the tumor, such as lymphocyte infiltration, macrophage phagocytosis, antigen presentation, and intercellular communication based on cytokines and exosomes. These interactions include immune surveillance and clearance to inhibit tumor growth, as well as the microenvironment that promotes tumor growth. Moreover, blocking or promoting these intercellular interactions has proven to be feasible and effective in clinical practice, such as vaccine therapy based on antigen-specific T cells, antibody therapy that relies on the cytotoxic effect of NK cell, and immune checkpoint inhibitor therapy for blocking tumor from immune escaping. In contrast, single-cell targeting drug delivery systems can only regulate physiological processes by aiming at specific target cells, but cannot utilize the intercellular interactions in tumor tissues to achieve effective treatment.

Therefore, in the present disclosure, antibodies against different cells are modified on a single liposome. The liposome with a stable structure and long circulation characteristics in vivo is constituted by molecular self-assembly. The liposome can sequentially or simultaneously bind to multiple different cells by means of the action of antibodies, to realize the blocking or promoting of intercellular interactions, so as to promote the recognition, communication, the cytotoxicity, the pro-apoptosis or clearance of different cells, etc. In addition, targeting drug delivery for multiple cells is also achieved by lipid drug carriers, which avoids problems such as drug interactions that may occur in combination therapy, and improves the pharmacological effects.

### Summary

The present disclosure provides a multicellular targeting liposome pharmaceutical preparation capable of sequentially or simultaneously binding to multiple different cells, and preparation and application thereof.

The objects of the present disclosure are achieved by the following technical solutions:

A first aspect of the present disclosure provides a multicellular targeting liposome. The multicellular targeting liposome includes a liposome, a predominant antibody and an auxiliary antibody modified on the surface of the liposome, the predominant antibody specifically binds to target molecules on the surface of the target cells, and the auxiliary antibody specifically binds to immune effector cells.

Further, the multicellular targeting liposome is capable of simultaneously or sequentially binding to immune effector cells and target cells, such that the immune effector cells recognize target cells and activate immune effects.

Further, the molar ratio of the predominant antibody to the auxiliary antibody on each liposome is between 100/1 and 1/1.

Further, each liposome has 1-1000 predominant antibodies and 1-1000 auxiliary antibodies on the surface.

Further, the predominant antibody and the auxiliary antibody are displayed on the surface of the liposome through covalent linkage or hydrophobic, hydrophilic interaction, respectively.

As exemplified in some embodiments of the present disclosure, the predominant antibody and the auxiliary antibody are respectively connected to a lipid molecule in the liposome. The lipid molecule has a maleimide group. The predominant antibody and the auxiliary antibody are respectively connected to the maleimide group of the lipid molecule. The predominant antibody and the auxiliary antibody are respectively connected to the lipid molecule via a thioether bond.

Further, the target cell is selected from a group consisting of a tumor cell, a microorganism, and a microorganism-infected cell. Therefore, the antigen targeted by the predominant antibodies is selected from a group consisting of a microbial antigen, a tumor-related antigen, a tumor cell surface specific antigen, a highly expressed antigen on the tumor cell surface, and a highly expressed antigen in a tumor tissue or a tumor blood vessel. That is, the target molecule is selected from a group consisting of a microbial antigen, a tumor-related antigen, a tumor cell surface specific antigen, a highly expressed antigen on the tumor cell surface, and a highly expressed antigen in a tumor tissue or a tumor blood vessel.

Further, the specific target molecule of the predominant antibody is selected from a group consisting of CD19, CD20, PSMA, Her2/neu, EGFR, LGR5 or PDL1.

In some embodiments of the present disclosure, the specific target molecule of the predominant antibody is a CD19. The CD19 is a human CD19 antigen of human B lymphocytic leukemia or human B cell lymphoma.

Further, the immune effector cell is an effector cell capable of producing cytotoxicity, pro-apoptosis or clearance effects on target cells. The immune effector cell is selected from T lymphocyte, NK cell, NKT cell, macrophage, or neutrophil.

Therefore, the antigen targeted by the auxiliary antibody is selected from the antigen expressed by tumor cells, lymphocytes or myeloid cells. Therefore, the multicellular targeting liposome can simultaneously or sequentially bind to a tumor cell, a lymphocyte, or a myeloid cell. That is, the same liposome first binds to a tumor cell and then a lymphocyte, or vice versa.

In some embodiments of the present disclosure, the immune effector cell is a T lymphocyte.

In some embodiments of the present disclosure, the effector antigen of the immune effector cell is CD3, and the auxiliary antibody is a CD3 antibody.

Further, the auxiliary antibody is selected from a CD3 antibody and a fragment, a PD1 antibody and a fragment, a CTLA4 antibody and a fragment, a CD40L antibody and a fragment.

The T lymphocyte may be an unactivated T cell, or an in-vitro activated or amplified T cell, for example, an antigen-specific T lymphocyte, a tumor infiltrating lymphocyte, a cytotoxic T cell, a helper T cell, a lymphokine-activated killer cell (LAK cells), a γδ T cell, a chimeric antigen receptor T cell, or a T cell receptor chimeric T cell.

The T lymphocyte is an unactivated T cell, or an in-vitro activated or amplified T cell.

The activation or amplification may be activation or amplification by antigen, antibody, polypeptide, polypeptide-major histocompatibility complex, small molecule, cytokine, immune checkpoint inhibitor or gene editing.

Further, the form of the predominant antibody and the auxiliary antibody is selected from IgG, Fab' fragment, F(ab')2 fragment, Fab fragment, single-chain Fv fragment, minibody, nanobody, unibody, or diabody.

As exemplified in some embodiments of the present disclosure, the antibody is a Fab' fragment of an antibody, which is obtained after removing the Fc fragment and reducing the antibody. The antibody includes a light chain variable region and a heavy chain variable region.

As exemplified in some embodiments of the present disclosure, the predominant antibody is a Fab' fragment of a CD19 antibody, and the amino acid sequence of the light chain variable region is shown in SEQ ID NO. 1, specifically:

DIQMTQTTSSLSASLGDRVTIS-
CRASQDIRNYLNWYQQKPDGTVKLLIYYTSRLHSGVPSKFSGSGSGTDYSLTI
SNLEQEDIATYFCQQGNTLPWTFAGGTKLEIK.

The amino acid sequence of the heavy chain variable region is shown in SEQ ID NO. 2, specifically:

EVQLQQSGPELVKPGASMKISCKASGYSFTGYTMNWVKQSHGKNLEW-
MGLINPYK-
GVSTYNQKFKDKATLTVDKSSSTAYMELLSLTSEDSAVYYCARSGYYGDSD
WYFDVWGQGTTLTVFS.

In addition, the predominant antibody may be a Her2 antibody, and the LC sequence of the Her2 antibody is shown in SEQ ID NO. 3, specifically:

MDMRVPAQLLGLLLLWLRGARCDIQMTQSPSSLSASVG-
DRVTITCRASQDVNTA-
VAWYQQKPGKAPKLLIYSASFLYSGVPSRFSGSRSGTDFTLTISSLQPEDFATY

YCQQHYTTPPTFGQGTKVEIKRTVAAPSVFIFPPSDEQLKSG-
TASVVCLLNNFYPREAK-
VQWKVDNALQSGNSQESVTEQDSKDSTYSLSSTLTLSKADYEKHKVYACEV
THQGLSSPVTKSFNRGEC.

The HC sequence of the Her2 antibody is shown in SEQ ID NO. 4, specifically:

MEFGLSWVFLVAILKGVQCEVQLVESGGGLVQPGGSLRLSCAASGFNIK-
DTYIHWVR-
QAPGKGLEWVARIYPTNGYTRYADSVKGRFTISADTSKNTAYLQMNSLRAED
TAVYYCSRWGGDGFYAMDYWGQGTLVTVSSASTKGPSVFPLAPSSKSTSGG-
TAALGCLVKDYFPEPVTVSWNSGALTSGVHTFPAVLQSSGLYSLSSVVTVPSS
SLGTQTYICNVNHKPSNTKVDKKV.

In addition, the predominant antibody may be an scFv fragment of an EGFR antibody, and the VH sequence of the scFv of the EGFR antibody is shown in SEQ ID NO. 5, specifically:

QVQLKQSGPGLVQPSQSLSITCTVSGFSLTNY-
GVHWVRQSPGKGLEWLGVIWS-
GGNTDYNTPFTSRLSINKDNSKSQVFFKMNSLQSNDTAIYYCARALTYYDYE
FAYWGQGTLVTVSA.

The VL sequence of the EGFR antibody is shown in SEQ ID NO. 6, specifically:

DILLTQSPVILSVSPGERVSFSCRASQSIGTNIHWYQQRTNGSPRLLIKYAS-
ESIS-
GIPSRFSGSGSGTDFTLSINSVESEDIADYYCQQNNNWPTTFGAGTKLELK.

The predominant antibody may be a PSMA antibody, and the VH sequence of the PSMA antibody is shown in SEQ ID NO. 7, specifically:

QVQLVESGGGLVKPGESLRLSCAASGFTFSDYYMYWVR-
QAPGKGLEWVAIISDG-
GYYTYYSDIIKGRFTISRDNAKNSLYLQMNSLKAEDTAVYYCARGFPLLRHG
AMDYWGQGTLVTVSS.

The VL sequence of the PSMA antibody is shown in SEQ ID NO. 8, specifically:

DIQMTQSPSSLSASVGDRVTITCKASQNVDTNVAWYQQKPGQAP-
KSLIYSASYRYSDVPSRFSGSASGTDFTLTISSVQSEDFATYYCQQYDSYPYTF
GGGTKLEIK.

The predominant antibody may be a PDL1 antibody, and the heavy chain sequence of the PDL1 antibody is shown in SEQ ID NO. 9, specifically:

EVQLLESGGG LVQPGGSLRL SCAASGFTFS SYIMMWVRQA PGKGLEWVSS
50
IYPSGGITFY ADTVKGRFTI SRDNSKNTLY LQMNSLRAED TAVYYCARIK
100
LGTVTTVDYW GQGTLVTVSS ASTKGPSVFP LAPSSKSTSG GTAALGCLVK
150
DYFPEPVTVS WNSGALTSGV HTFPAVLQSS GLYSLSSVVT VPSSSLGTQT
200
YICNVNHKPS NTKVDKKVEP KSCDKTHTCP PCPAPELLGG PSVFLFPPKP
250
KDTLMISRTP EVTCVVVDVS HEDPEVKFNW YVDGVEVHNA
KTKPREEQYN 300
STYRVVSVLT VLHQDWLNGK EYKCKVSNKA LPAPIEKTIS KAKGQPREPQ
350
VYTLPPSRDE LTKNQVSLTC LVKGFYPSDI AVEWESNGQP ENNYKTTPPV
400
LDSDGSFFLY SKLTVDKSRW QQGNVFSCSV MHEALHNHYT QKSLSLSPGK
450.

The light chain sequence of the PDL1 antibody is shown in SEQ ID NO. 10, specifically:

QSALTQPASV SGSPGQSITI SCTGTSSDVG GYNYVSWYQQ HPGKAPKLMI
50
YDVSNRPSGV SNRFSGSKSG NTASLTISGL QAEDEADYYC SSYTSSSTRV
100
FGTGTKVTVL GQPKANPTVT LFPPSSEELQ ANKATLVCLI SDFYPGAVTV
150
AWKADGSPVK AGVETTKPSK QSNNKYAASS YLSLTPEQWK SHRSYSCQVT

200
HEGSTVEKTV APTECS.

As exemplified in some embodiments of the present disclosure, the auxiliary antibody is a Fab' fragment of a CD3 antibody, and the VH sequence of the CD3 antibody is shown in SEQ ID NO. 11, specifically:

DIKLQQSGAELARPGASVKM-
SCKTSGYTFTRYTMHWVKQRPGQGLEWIGYINPSR-
GYTNYNQKFKDKATLTTDKSSSTAYMQLSSLTSEDSAVYYCARYYDDHYCL
DYWGQGTTLTVSS.

The VL sequence of the CD3 antibody is shown in SEQ ID NO. 12, specifically:

DIQLTQSPAIMSASPGEKVTMTCRASSSVSYMNWYQQKSGTSPKRWIYDTS-
KVASGVPYRFSGSGSGTSYSLTISSMEAEDAATYYCQQWSSNPLTFGAGTKLE
LK.

Alternatively, the VH sequence of the CD3 antibody is shown in SEQ ID NO. 13, specifically:

EVQLVESGGGLVQPGGSLKLSCAASGFTFNKYAMNWVR-
QAPGKGLEWVARIRSKYNNYATY-
YADSVKDRFTISRDDSKNTAYLQMNNLKTEDTAVYYCVRHGNFGNSYISYW
AYWGQGTLVTVSS.

The VL sequence of the CD3 antibody is shown in SEQ ID NO. 14, specifically:

QTVVTQEPSLTVSPGGTVTLTCGSSTGAVTSGNYPNWVQQKPGQAPR-
GLIGGTKFLAPGT-
PARFSGSLLGGKAALTLSGVQPEDEAEYYCVLWYSNRWVFGGGTKLTVLA.

The auxiliary antibody may also be a PD-1 antibody, and the VH sequence of the PD-1 antibody is shown in SEQ ID NO. 15, specifically:

-
-

.

The VL sequence of the PD-1 antibody is shown in SEQ ID NO. 16, specifically:

LEMAEIVLTQSPATLSLSPGERATLSCRASQSVSSYLAWYQQKPGQAPRLLI-
YDASNRAT-
GIPARFSGSGSGTDFTLTISSLEPEDFAVYYCQQSSNWPRTFGQGTKVEIK.

The present disclosure has no special limitation on the composition of the liposome, as long as it is a liposome. The components of the liposome may be selected from the group consisting of egg phospholipid, hydrogenated soybean phosphatidylcholine, hydrogenated egg phosphatidylcholine, dilauroylphosphatidylcholine, dimyristoylphosphatidylcholine, dipalmitoylphosphatidylcholine, distearoylphosphatidylcholine, 1-myristoyl-2-palmitoylphosphatidylcholine, 1-palmitoyl-2-stearoylphosphatidylcholine, 1-stearoyl-2-palmitoylphosphatidylcholine, 1-palmitoyl-2-oleylphosphatidylcholine, 1-stearoyl-2-linoleylphosphatidylcholine, dioleylphosphatidylcholine, hydrogenated dipalmitoylphospholipidcholine, distearoylphosphatidylcholine, dimyristoylphosphatidic acid, dimyristoylphosphatidic acid, dipalmitoylphosphatidic acid, dipalmitoylphosphatidic acid, distearoylphosphatidic acid, dimyristoyl phosphatidylethanolamine, dipalmitoylphosphatidylethanolamine, brain phosphatidylserine, dimyristoylphosphatidylserine, dipalmitoylphosphatidylserine, egg phosphatidylglycerol, dilauroylphosphatidyl glycerol, dimyristoylphosphatidyl glycerol, dipalmitoylphosphatidylglycerol, distearylphosphatidylglycerol, dioleoylphosphatidylglycerol, brain sphingomyelin, dipalmitoyl sphingomyelin or distearoyl sphingomyelin, cholesterol, dioleoxypropyltrimethylammonium chloride (DOTAP), dioleyl chloropropyl trimethylammonium chloride (DOTMA), dimethyl-dioctadecylammonium bromide (DDAB), dimethylaminoethane carbamoyl-cholesterol (DC-Chol), spermine-5- carboxyaminoacetic acid octacosylamide (DOGS), di-oleosuccinylglycerol choline ester (DOSC), dioleyl chlorospermine carboxyl amide ethyl dimethyl propyl ammonium trifluoroacetate (DOSPA), or a combination of two or more thereof.

As exemplified in some embodiments of the present disclosure, the components of the liposome include phosphatidylcholine, cholesterol, a lipid linked to a predominant antibody, and a lipid molecule linked to an auxiliary antibody.

Further, the liposome may be a blank liposome or a drug-loading liposome.

A second aspect of the present disclosure provides a method for preparing the aforementioned multicellular targeting liposome. A post-insertion method is used. The method includes the following:
(1) connecting the predominant antibody and the auxiliary antibody with the lipid molecule respectively to obtain the predominant antibody-lipid molecule and the auxiliary antibody-lipid molecule;
(2) mixing the obtained predominant antibody-lipid molecule and the auxiliary antibody-lipid molecule with the constructed liposome, and incubating to obtain a mixed solution;
(3) dialyzing or ultrafiltrating the obtained mixed solution to remove unloaded antibody and antibody-lipid complexes, and obtaining the multicellular targeting liposome.

Preferably, the molar ratio of the predominant antibody to the lipid molecule is (0.1-5): 1000.

Preferably, the molar ratio of the auxiliary antibody to the lipid molecule is (0.1-5): 1000.

A third aspect of the present disclosure provides the use of the aforementioned multicellular targeting liposome in preparing immunotherapeutic drugs and antitumor drugs.

A fourth aspect of the present disclosure provides a method for treating tumors, including: administering the aforementioned multicellular targeting liposome to a tumor patient. The method may be in vitro. The method may be non-therapeutic. The tumor may be a tumor that is CD19-positive on the cell surface.

Compared with the traditional technology, the present disclosure has the following beneficial effects:
The multicellular targeting liposome of the present disclosure has a mechanism similar to that of the multi-targeting antibodies. The present disclosure further proposes to optimize effector cell activation effects on different target cells and target molecules by adjusting the density and relative proportions of the predominant antibody and auxiliary antibody. Furthermore, the effects of drugs loaded in the liposome on target cells and effector cells are further enhanced. Finally, the production, preparation and quality control of the multicellular liposome are also more feasible compared with multi-targeting antibodies.

### Brief Description of the Drawings

FIG. 1A: Reduced SDS-PAGE images and non-reduced SDS-PAGE images of full-length CD3 antibody, purified anti-CD3 antibody F(ab')2 fragment, anti-CD3 antibody Fab' fragment-polymer-DSPE linked product, stained with Coomassie brilliant blue.
FIG. 1B: SDS-PAGE images of full-length CD19 antibody, purified anti-CD19 antibody F(ab')2 fragment, anti-CD19 antibody Fab' fragment-polymer-DSPE linker, stained with Coomassie brilliant blue.
FIG. 1C: SDS-PAGE images of anti-CD3 single-chain antibody (scFv) fragment, anti-CD3 single-chain antibody (scFv) fragment-polymer-DSPE linker, stained with Coomassie brilliant blue.
FIG. 2: Particle size distribution of multicellular targeting liposome, single-targeting liposome, and non-targeting liposome.
FIG. 3A: The specific binding of CD3 and CD19 multicellular targeting liposome and single-targeting liposome to target cells Jurkat.
FIG. 3B: The specific binding of CD3 and CD19 multicellular targeting liposome and single-targeting liposome to target cells Raji.
FIG. 4: Intercellular binding of CD3+ and CD19+ target cells mediated by multicellular targeting liposome, single-targeting liposome, and non-targeting liposome.
FIG. 5A: The cell-killing effect of the preactivated effector cells mediated by the multicellular targeting liposome on target cells Raji; the cytotoxicity 24 h after dosing is detected by the LDH method; the effector cells are T cells after activation and amplification by CD3 antibody and CD28 antibody for 3 days.
FIG. 5B: The cell-killing effect of the preactivated effector cells mediated by the multicellular targeting liposome on target cells Raji; the cytotoxicity 24 h after dosing is detected by the LDH method; the effector cells are LAK cells after activation and amplification by human IL-2.
FIG. 6A: The cell-killing effect of the preactivated effector cells mediated by the multicellular targeting liposome on target cells Raji, the effector cells are human γδT cells after amplification by zoledronic acid; the cytotoxicity 24 h after dosing is detected by LDH method; cell killing situation with different effector cell/target cell ratios.
FIG. 6B: The cell-killing effect of the preactivated effector cells mediated by the multicellular targeting liposome on target cells Raji, the effector cells are human γδT cells after amplification by zoledronic acid; the cytotoxicity 24 h after dosing is detected by LDH method; cell killing situation mediated by liposomes with different dose and targeting.
FIG. 6C: The cell-killing effect of the preactivated effector cells mediated by the multicellular targeting liposome on target cells Raji, the effector cells are human γδT cells after amplification by zoledronic acid; the cytotoxicity 24 h after dosing is detected by LDH method; cell killing situation mediated by liposomes with different dose and targeting.
FIG. 7A: The cell-killing effect of the unstimulated effector cells mediated by the multicellular targeting liposome on target cells Raji, the effector cells are unactivated human lymphocytes; the cytotoxicity 5 h after dosing is detected by PI staining method, i.e. the target cell killing situation after 5 h of dosing.
FIG. 7B: The cell-killing effect of the unstimulated effector cells mediated by the multicellular targeting liposome on target cells Raji, the effector cells are unactivated human lymphocytes; the cytotoxicity 24 h after dosing is detected by propidium iodide (PI) staining method, i.e. the target cell killing situation after 24 h of dosing.
FIG. 8A: The cell-killing effect of the unstimulated lymphocyte mediated by the multicellular targeting liposome on target cells Raji, the effector cells are unactivated human lymphocytes; cell killing situation with different effector cell/target cell ratios (PI staining method).
FIG. 8B: The cell-killing effect of the unstimulated lymphocyte mediated by the multicellular targeting liposome on target cells Raji, the effector cells are unactivated human lymphocytes; cell killing situation mediated by liposomes with different dose and targeting. (LDH method).
FIG. 8C: The cell-killing effect of the unstimulated lymphocyte mediated by the multicellular targeting liposome on target cells Raji, the effector cells are unactivated human lymphocytes; cell killing situation mediated by liposomes with different dose and targeting. (LDH method).
FIG. 9A: The cell-killing effect of the unstimulated lymphocyte mediated by the multicellular targeting liposome on target cells, the effector cells are unactivated lymphocytes, and the target cells are Raji cells; the cytotoxicity 5 h after dosing is detected by LDH method. The predominant antibody of the multicellular targeting liposome is a different clone of the anti-CD3 IgG monoclonal antibody (2C11) instead of the Fab'2 fragment of the anti-CD3 antibody (UCHT1 clone).
FIG. 9B: The cell-killing effect of the unstimulated lymphocyte mediated by the multicellular targeting liposome on target cells; the effector cells are unactivated human lymphocytes, and the target cells are CD19 + Human B lymphocytic leukemia SUP-B15 cells; the cytotoxicity 24 h after dosing is detected by LDH method.
FIG. 9C: The cell-killing effect of the unstimulated lymphocyte mediated by the multicellular targeting liposome on target cells; the effector cells are unactivated CD4+T lymphocytes, and the target cells are LGR5+ human non-small cell lung cancer A549 cells; the cytotoxicity 5 h after dosing is detected by LDH method; the auxiliary antibody of the multicellular targeting liposome is the LGR5 antibody.
FIG. 10: Secretion of IFN-γ under the cell-killing effect of the unstimulated lymphocyte mediated by the multicellular targeting liposome on target cells Raji cells; the effector cells are unactivated human lymphocytes, PxP refers to the non-targeting liposome group, HxP refers to the CD19 single-targeting liposome group, UxP refers to the CD3 single-targeting liposome group, UxH refers to the CD19 and CD3 multicellular targeting liposome group.
FIG. 11: Under the cell-killing effect of the unstimulated lymphocyte mediated by the multicellular targeting liposome on target cells Raji, the impact of different concentrations of EGTA; the effector cells are unstimulated human lymphocytes.
FIG. 12: The cell-killing effect of the unstimulated lymphocyte mediated by the multicellular targeting liposome on target cells; the effector cells are unstimulated human lymphocytes, and the target cells are CD19+ Raji cells; the cytotoxicity 24 h after dosing is detected by LDH method; UxH-lipo refers to the multicellular targeting blank liposome group, UxH-DAS-lipo refers to the multicellular targeting dasatinib liposome group.

### Detailed Description of the Preferred Embodiments

Liposomes are artificially prepared bilayer vesicles formed by lipid molecules. The structure of a lipid molecule usually consists of a hydrophilic head, a hydrophobic tail, and a link section. In the aqueous medium, due to hydrophobic interactions, the hydrophobic tails of the lipid molecules are close to each other, and the hydrophilic heads are arranged facing toward the aqueous phase, forming a bilayer. Based on such a structure, hydrophilic drugs can be embedded in the internal aqueous phase (internal water phase) of the liposome, and hydrophobic drugs can be embedded in the lipid bilayer.

The targeting effect of the targeting liposome is achieved by modifying the ligand on the surface. The ligand may be an antibody, a polypeptide, a small molecule, or the like. The ligand interacts with the target cell specific receptor. The antibody and antibody fragments are modified on the surface of the liposome, and have better binding activity and specificity compared with small molecules and polypeptide ligands. Applying the principle of self-assembly of lipid molecules, it is also feasible to modify two or more different ligands on the surface of one liposome.

Based on the antibody-modified liposome technology, the present disclosure, on the one hand, obtains a novel and unique killing mechanism and effect of liposome on tumor cells through a clever combination of a predominant antibody against a target cell and an auxiliary antibody against an effector cell. The currently known multi-targeting liposome preparation technologies use the combination of different ligands to combine different antigens, to improve the targeting delivery efficiency and selectivity of specific cells. The multicellular targeting liposome of the present disclosure regulates physiological functions such as recognition, binding, and activation between different cells by promoting or blocking interactions between various cells. Therefore, the present disclosure provides a new direction for ligand-targeting liposome technology.

On the other hand, constructing multicellular targeting ability on the surface of a lipid drug carrier is not only more convenient than constructing two antibody sequences with different targeting ability on the same antibody structure, but also can optimize the anti-tumor effect by adjusting the density and proportion of the predominant antibody and auxiliary antibody on the surface of the liposome. In addition, such construction and preparation of the multicellular targeting liposome would also avoid problems such as antibody mismatch and overcome the defects in production and efficacy. In particular, the liposome has unique pharmacokinetic and tissue distribution characteristics in vivo, which greatly improve the defects of short in-vivo half-life of current multi-targeting antibodies.

Further, the multicellular targeting liposome obtained by the present disclosure may combine with liposome drug-loading technology, which is more convenient, diverse and controllable compared with the traditional clinically available combination therapy of multi-targeting antibody and other drugs. By encapsulating the drug in the liposome, the targeting delivery and release of the drug is achieved, that is, the selectivity of the drug is improved, and the releasing site and time of the drug can be changed. For example, the adverse effects of tumor immunotherapy include the production of cytokine storms in patients, so patients are clinically required to take dexamethasone in advance to suppress the adverse reaction. For another example, some immune drugs require systemic administration of cytokine drugs such as IL-2 to achieve the desired immune activation effect. Liposomes, as drug delivery carriers, can achieve targeting delivery of drugs by encapsulating drugs and using predominant antibodies and auxiliary antibodies.

Therefore, by preparing a multicellular targeting liposome including the predominant antibody against the target cell and the auxiliary antibody against the effector cell, the present disclosure can promote the immune cells to bind and recognize tumor cells, and then activate the effector function of the immune cells. The multicellular targeting liposome has a unique mechanism of in-vivo action, and has substantial progress in production, preparation and in-vivo effects compared with the clinically applied multi-targeting antibodies. In addition, the drug-loading liposome may be used to prepare multicellular targeting preparations to achieve better therapeutic effects.

As exemplified in some embodiments of the present disclosure, the technical solution of the present disclosure has the following advantages:
(1) the present disclosure starts from the preparation of a multicellular targeting liposome, modifying two targeting antibodies or antibody fragments with different specificities on the same liposome. The liposome preparation and surface targeting modification are mature in technology, simple in design, and are easy for large-scale production and isolation and purification. The targeting group may be adjusted according to clinical needs, which is conducive to the development and design of various multicellular targeting combinations with different specificities according to the disease.
(2) two antibody fragment-lipid linkers specifically binding to CD19 antigen and CD3 antigen, and the multicellular targeting liposome and single-targeting liposome prepared with fluorescently labeled liposomes show strong binding activity when incubating with CD19 positive and CD3 positive cells, respectively, and such binding is specific;
(3) the multicellular targeting liposomes specifically binding to the CD19 antigen and CD3 antigen are co-incubated with CD19-positive and CD3-positive cells with different fluorescent labels, and two kinds of fluorescent double-positive signals are detected by flow cytometry, showing that multicellular targeting liposomes can effectively mediate the intercellular binding, i.e. the ability to promote immune effector cells to recognize the target cells;
(4) the multicellular targeting liposomes specifically binding to the CD19 antigen and CD3 antigen are co-incubated with immune effector cells and tumor cell, the results show that the immune effector cells have a strong ability to kill tumor cells, and this killing is mediated by the perforin/granzyme pathway;
(5) the multicellular targeting liposomes specifically binding to the CD19 antigen and CD3 antigen are co-incubated with immune effector cells and tumor cell, the ELISA results show that multicellular targeting liposomes can effectively stimulate the secretion of IFN-γ cytokines from immune effector cells;
(6) according to LDH test, the multicellular targeting liposomes specifically binding to the CD 19 antigen and CD3 antigen show that the multicellular targeting liposomes have the controllable ability to regulate the cell interaction by regulating the density of the antibodies on the surface;
(7) the multicellular targeting liposomes specifically binding to the CD19 antigen and CD3 antigen not only have the advantages of multi-targeting antibodies, but also can use drug-loading liposomes to prepare multicellular targeting drug delivery systems. By using the two antibody fragment-lipid linkers that specifically bind to CD19 antigen and CD3 antigen and drug-loading liposome loaded with immunoregulatory drug dasatinib as the raw materials, the prepared drug-loading multicellular targeting liposomes show effective inhibiting capacity against immune effector-mediated cell killing under high drug loading. The drug-loading multicellular targeting liposomes may serve as targeting drug carriers to develop into targeting drug delivery systems for diseases and achieve better therapeutic effects.

Before further describing the specific embodiments of the present disclosure, it should be understood that the scope of protection of the present disclosure is not limited to the following specific embodiments; it should also be understood that the terms used in the embodiments of the present disclosure are just for describing the specific embodiments instead of limiting the scope of the present disclosure. The test methods without specific conditions noted in the following embodiments are generally based on conventional conditions or the conditions recommended by the manufacturers.

When the numerical ranges are given by the embodiments, it is to be understood that the two endpoints of each numerical range and any value between the two may be selected unless otherwise stated. Unless otherwise defined, all technical and scientific terms used in the present disclosure have the same meaning as commonly understood by one skilled in the art. In addition to the specific method, equipment and material used in the embodiments, any method, equipment and material in the existing technology similar or equivalent to the method, equipment and material mentioned in the embodiments of the present disclosure may be used to realize the invention according to the grasp of the existing technology and the record of the invention by those skilled in the art.

Unless otherwise stated, the experimental methods, detection methods, and preparation methods disclosed in the present invention all employ conventional techniques of molecular biology, biochemistry, chromatin structure and analysis, analytical chemistry, cell culture, recombinant DNA technology in the technical field and related fields. These techniques are well described in the prior literature. For details, please see Sambrook et al. MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolfe, CHROMATIN STRUCTURE AND FUNCTION, Third Edition, Academic Press, San Diego, 1998; METHOD IN ENZYMOLOGY, Vol. 304, Chromatin (PMWassarman and APWolffe, eds.), Academic Press, San Diego, 1999; and METHODS IN MOLECULAR BIOLOGY, Vol. 119, Chromatin Protocols (PBBecker, ed.) Humana Press, Totowa, 1999, etc.

Unless otherwise specified, the experimental materials used in the following embodiments were purchased from conventional biochemical reagent stores.

### Experimental reagents and cells

Lecithin (EPC), hydrogenated soybean phosphatidylcholine (HSPC) and distearoyl phosphatidylethanolamine-polyethylene glycol 2000-maleimide (DSPE-PEG2000-mal) were purchased from NOF Corporation (Tokyo, Japan). Cholesterol, distearoyl phosphatidylethanolamine-polyethylene glycol 2000 (DSPE-PEG2000) were purchased from Avanti Polar Lipids (AL, US).

DiI was purchased from Sigma and CFSE was purchased from Invitrogen.

RPMI-1640 medium, fetal calf serum, penicillin-streptomycin double-antibody reagent (100 ×) were purchased from Gibico.

Raji human lymphoma cells and Jurkat human lymphoma cells were purchased from the Institute of Cells, Chinese Academy of Sciences.

### Embodiment 1 Preparation of multicellular targeting liposome

### 1. Preparation of F(ab')2 fragments of anti-CD19 antibody and anti-CD3 antibody

The F(ab')2 fragment is prepared by excising the Fc fragment of IgG by the digestion reaction of an immobilized enzyme (pepsin or ficin). The human IgG and mouse IgG are digested by pepsin, and mouse IgG1 is digested by ficin. The specific steps are as follows: centrifuging 500 µl of antibody, and passing through a desalting column; adding the flow-through liquid to the immobilized enzyme, and mixing at 37 °C; incubating the pepsin for 4h or incubating the ficin for 24 h, collecting the flow-through liquid by centrifuging at 5,000 g for 1 min, washing the immobilized enzyme with Protein A Binding Buffer and collecting the flow-through liquid, and merging the flow-through liquids to obtain the enzyme-digested product.

Mixing the enzyme-digested product at a constant temperature in Protein A column for 10 min, collecting the flow-through liquid by centrifuging, washing the protein A column with Protein A Binding Buffer and collecting the flow-through liquid. Merging the flow-through liquids to obtain the enzyme-digested product purified by Protein A column and with the large fragments of IgG and Fc removed. Dialyzing the above enzyme-digested product through a 50KD dialysis bag, and the dialysis medium is PBS solution (pH = 7.0). The dialysis is performed in the order of 2h, 2h and 16h to obtain a purified antibody F(ab')2 fragment with the Fc fragment removed.

It is confirmed by the traditional technology that the obtained anti-CD 19 antibody F(ab')2 fragment is the full-length anti-CD 19 antibody with Fc fragment being removed.

It is confirmed by the traditional technology that the obtained anti-CD3 antibody F(ab')2 fragment is the full-length anti-CD3 antibody with the Fc fragment being removed. A light chain variable region and a heavy chain variable region are included. The amino acid sequence of the light chain variable region is shown in SEQ ID NO. 1, specifically:

DIQMTQTTSSLSASLGDRVTIS-
CRASQDIRNYLNWYQQKPDGTVKLLIYYTSRLHSGVPSKFSGSGSGTDYSLTI
SNLEQEDIATYFCQQGNTLPWTFAGGTKLEIK.

The amino acid sequence of the heavy chain variable region is shown in SEQ ID NO. 2, specifically:

EVQLQQSGPELVKPGASMKISCKASGYSFTGYTMNWVKQSHGKNLEW-
MGLINPYK-
GVSTYNQKFKDKATLTVDKSSSTAYMELLSLTSEDSAVYYCARSGYYGDSD
WYFDVWGQGTTLTVFS.

### 2. Preparation Fab' fragment (of anti-CD19 antibody and anti-CD3 antibody)-polymer-lipid linker

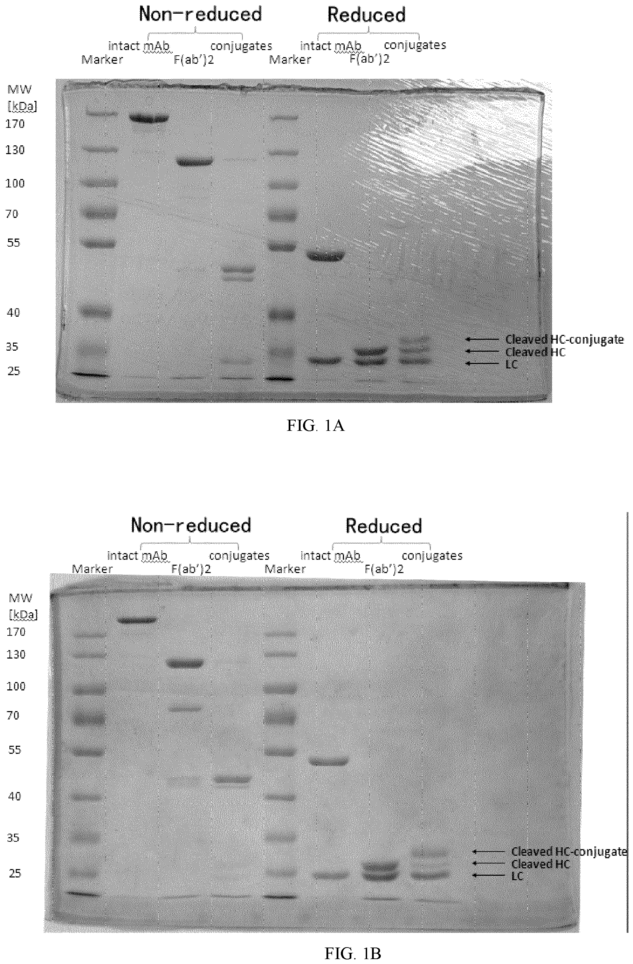

The antibody F(ab')2 fragment is reduced to Fab' fragment by using β-mercaptoethylamine as a reducing agent. The Fab' fragment is chemically linked to the maleimide group of DSPE-PEG2000-Mal. The specific steps are as follows: mixing the F(ab') 2 fragment (1-10 mg/ml) with 50 mM EDTA-containing β-mercaptoethylamine, reacting at 37 °C under the protection of N₂ with shaking for 90 min; centrifuging, passing through a desalting column and collecting the flow-through liquid. Fab', the reduction product of the antibody F(ab')2 fragment from which β-mercaptoethylamine is removed, is obtained. Then, adding 30mM HEPES solution containing 600uM DSPE-PEG2000-Mal micelle, so that the molar ratio of Fab' to DSPE-PEG2000-Mal is 1: 1, and the reaction is performed at 10 °C under the protection of N₂ with shaking for 16 h. The antibody ab' fragment-polymer-lipid linker, i.e. DSPE-PEG2000-Mal-Fab' micelle, is obtained. The linking situation of the obtained linker product is examined by SDS-PAGE. The results are shown in FIGs. 1A and 1B.

### 3. Preparation of anti-CD3 antibody scFv fragment-polymer-lipid linker

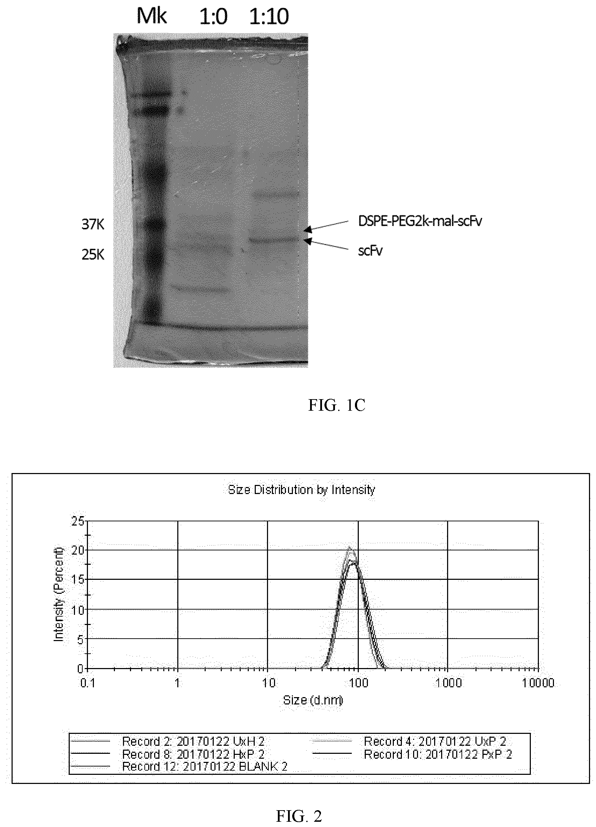

The antibody scFv fragment and its multimer are reduced to scFv containing free sulfydryl group by using tricarboxyethyl phosphine (TCEP) as a reducing agent, and chemically linked to the maleimide group of DSPE-PEG2000-Mal. The specific steps are as follows: mixing 800 µl of scFv fragment (1-10 mg/ml) with HEPES solution containing 1.2 mM TCEP, so that the molar ratio of scFv to TCEP is 1: 5, then adding 30mM HEPES solution containing 600 µM DSPE-PEG2000-Mal micelle immediately, and reacting at 10 °C under the protection of N₂ with shaking for 16 h; The antibody scFv fragment-polymer-lipid linker, i.e. DSPE-PEG2000-Mal-scFv micelle, is obtained. The linking situation of the obtained linker product is examined by SDS-PAGE. The result is shown in FIG. 1C.

### 4. Preparation of anti-CD19 and anti-CD3 single-targeting and multicellular targeting liposomes

The single-targeting and multicellular targeting liposomes are prepared by the post-insertion method. For multicellular targeting liposome, the prepared anti-CD19 and anti-CD3 Fab' fragment-polymer-lipid linker (DSPE-PEG2000-Mal- Fab') or scFv fragment-polymer-lipid linker is mixed with liposome in a molar ratio (0.1-5): (0.1-5): 1000, and the reaction is performed at 37 °C under the protection of N₂ with shaking for 24 h. Then, the liposomes are washed 5 times by 300KD ultrafiltration to remove the unlinked antibody Fab' fragments to obtain anti-CD19 and anti-CD3 multicellular targeting liposomes. For anti-CD3 single-targeting liposomes, the anti-CD19 Fab' fragment-polymer-lipid linker DSPE-PEG2000-Mal-Fab' is replaced with equimolar DSPE-mPEG2000. For anti-CD19 single-targeting liposomes, the anti-CD3 Fab' fragment-polymer-lipid linker DSPE-PEG2000-Mal-Fab' is replaced with equimolar DSPE-mPEG2000. The results are shown in FIG. 2 and Table 1: The particle size of the prepared targeting liposomes is about 80-90 nm, and the PDI is about 0.1.

**Table 1**

| | Z-AVE (nm) | PDI |
|---|---|---|
| blank | 80.3±1.5 | 0.06±0.01 |
| aCD3xaCD19 | 89.0±2.3 | 0.08±0.01 |
| aCD3xmPEG | 84.1±3.2 | 0.11±0.04 |
| aCD19xmPEG | 83.2±0.8 | 0.08±0.02 |
| mPEGxmPEG | 82.8±3.6 | 0.10±0.01 |

### Embodiment 2 Evaluation of in-vitro specific binding effect of multicellular targeting liposome

CD19-positive Raji cells and CD3-positive Jurkat cells are used as representatives to investigate the in-vitro specific binding effect of multicellular targeting liposomes.

Jurkat, Clone E6-1 human T lymphocytic leukemia cell line and Raji human B cell lymphoma cell line were purchased from the Cell Bank of the Chinese Academy of Sciences. The cell strain passes through a complete medium of RPMI-1640 (GIBCO) containing 1% pls double antibody + 10% FBS.

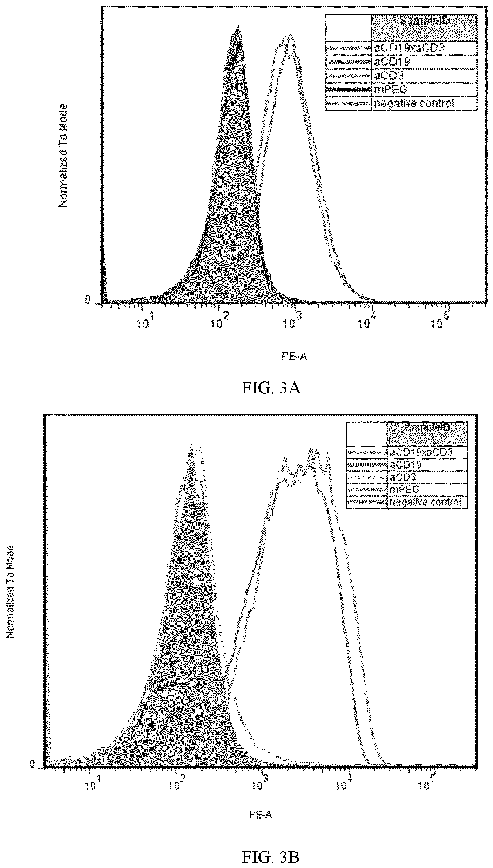

CD3-positive cell line Jurkat cells, CD19-positive cell line Raji cells, and human peripheral blood lymphocytes are analyzed by flow cytometry to examine the in-vitro specific binding capacity of the multicellular targeting liposome. 1 × 10⁶ Raji or Jurkat cells are resuspended in 400 µl complete medium, added to a 48-well plate, with lug single-targeting DiI labeled liposomes or multicellular targeting DiI labeled liposomes added to each well. Incubating in a cell incubator at 37 °C for 2 h. Then removing the cells and placing in a flow tube, centrifuging at 500 × g for 5 min, discarding the supernatant, and washing the cells twice with PBS. Resuspending the cells with 400 µl PBS and detecting the sample by flow cytometry Becton Dickinson FACS LSRII. The results are shown in FIGs. 3A and 3B.

### Embodiment 3 Evaluation of intercellular binding effect of target cells mediated by multicellular targeting liposome

CD19-positive Raji cells and CD3-positive Jurkat cells are used as representatives to demonstrate that the multicellular targeting liposome can mediate the cell-cell binding between different target cells.

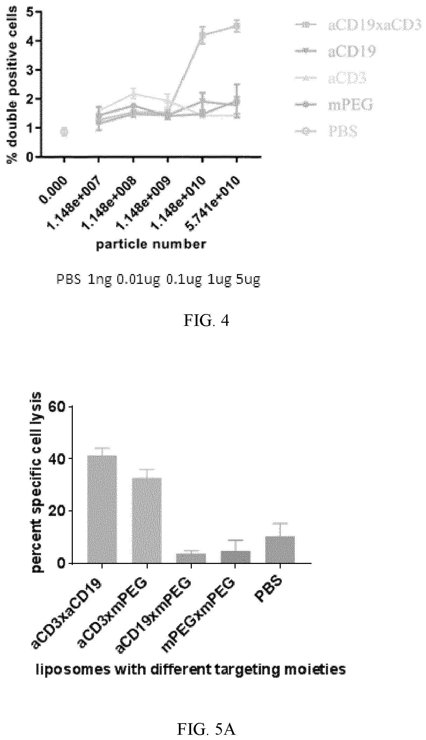

Jurkat cells are stained by CFSE, and Raji cells are stained by DiI. 1.25 × 10⁵ Raji-DiI and 1.25 × 10⁵ Jurkat-CFSE cells are resuspended in 400 µl phenol red-free complete medium, mixed and added to a 48-well plate, with 1 µg single-targeting liposomes or multicellular targeting liposomes added to each well. Incubating in a 37 °C cell incubator for 30 min. CFSE-positive Jurkat cells, DiI-positive Raji cells, and CFSE/DiI double-positive cell clusters are analyzed by flow cytometry to examine the intercellular binding effect of target cells mediated by multicellular targeting liposomes. The result is shown in FIG. 4.

### Embodiment 4 Evaluation of the killing effect of effector cells mediated by multicellular targeting liposome on tumor cells

The cell culture medium is formulated by adding 5% FBS, 1% double antibody, and 100 IU/ml hIL-2 to phenol red-free RPMI-1640 medium.

### 1. Evaluation of tumor cell killing effect by LDH method

The killing effect of effector cells mediated by multicellular targeting liposome on tumor cells is detected by CytoTox 96® non-radioactive cytotoxicity test kit (Promega Co.).

Effector cells (unstimulated human lymphocytes, CD3/CD28 antibody amplified and activated human T cells, γδ T cells, LAK cells) and target cells (Raji cells, SIP-B15 cells, A549 cells) are resuspended in phenol red-free complete medium, and the cell concentrations are 1^{∗}10^6 cells/ml and 1.11^{∗}10^5 cells/ml, respectively. Adding 100 µl effector cells and 90 µl target cells to a 96-well plate and mixing by pipetting to plate the cells. At this time, the ratio of target cells to effector cells is 1:10. Adding 10 µl of targeting liposomes of different concentrations to each well, and mixing by pipetting. Placing the 96-well plate in a cell incubator, incubating for 24 h. 45 min before the end of the incubation, adding 10 µl lysate to the volume-correcting control well and the targeting cell maximum lysis well. After the incubation, centrifuging the 96-well plate at 250 g for 4 min. After centrifugation, pipetting 50 µl of the supernatant to a new 96-well plate, adding 50µl of LDH substrate to the new well plate, and incubating at 22 °C for 15 min. After incubation, detecting the absorbance in a microplate reader at a wavelength of 490 nm.

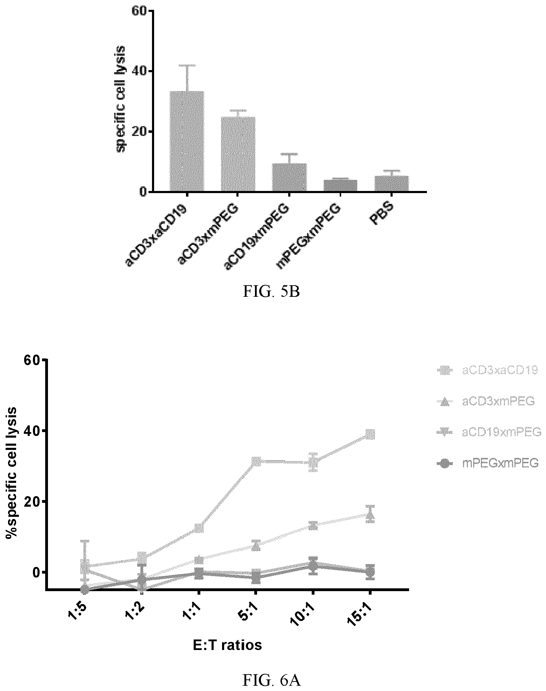

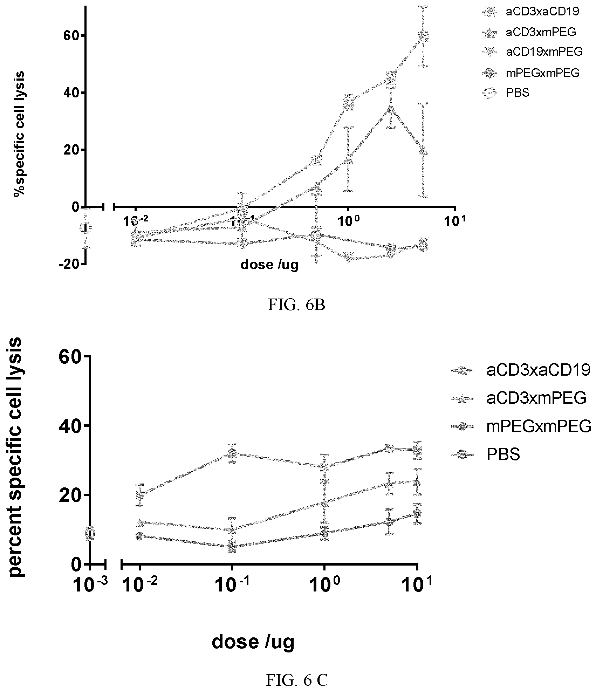

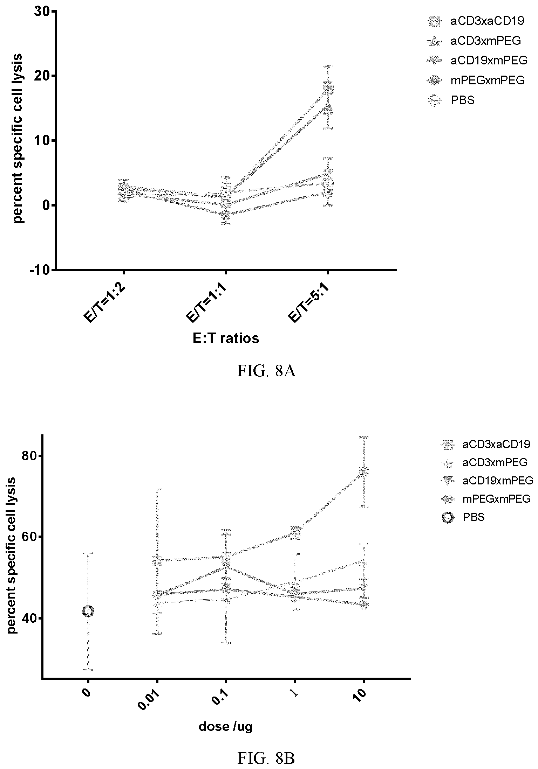

The results are shown in FIGs. 5A, 5B, 6A, 6B, 6C, 8B, 8C, and 9.

These experimental data fully demonstrate that the multicellular targeting liposome of the present disclosure can effectively mediate the effector cells to effectively kill the target cells, and the effector cells may include unstimulated lymphocytes, in-vitro amplified lymphocytes, in-vitro activated lymphocytes, in-vitro induced differentiated lymphocytes. The cytotoxic effect is dependent on cell lysis and is dose-dependent.

### 2. Evaluation of tumor cell killing effect by PI staining method

Effector cells (unstimulated human lymphocytes, CD3/CD28 antibody amplified and activated human T cells, γδ T cells, LAK cells) and CFSE-labeled target cells (Raji cells) are resuspended in phenol red-free complete medium, and the cell concentrations are 1^{∗}10^6 cells/ml and 1.11^{∗}10^5 cells/ml, respectively. Adding 100 µl effector cells and 90 µl target cells to a 96-well plate and mixing by pipetting to plate the cells. At this time, the ratios of target cells to effector cells are 1:1, 1:5, and 1:10. Adding 10 µl of targeting liposomes of different concentrations to each well, and mixing by pipetting. Place the 96-well plate in a cell culture incubator for 24 h. After the incubation, collecting the cell suspension into a flow tube, adding 5 µl 1mg/ml PI, incubating for 15 min, then adding 200 µl of flow buffer and loading the sample.

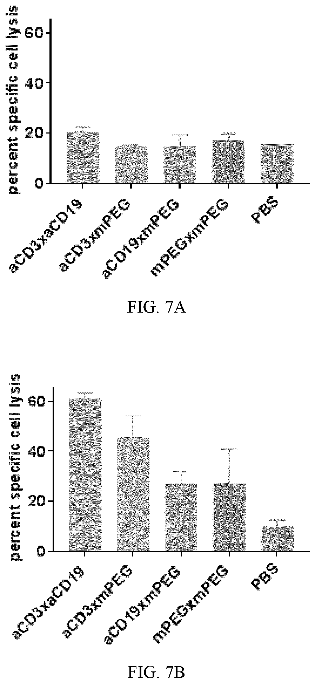

The results are shown in FIGs. 7A, 7B, 8A and 9B.

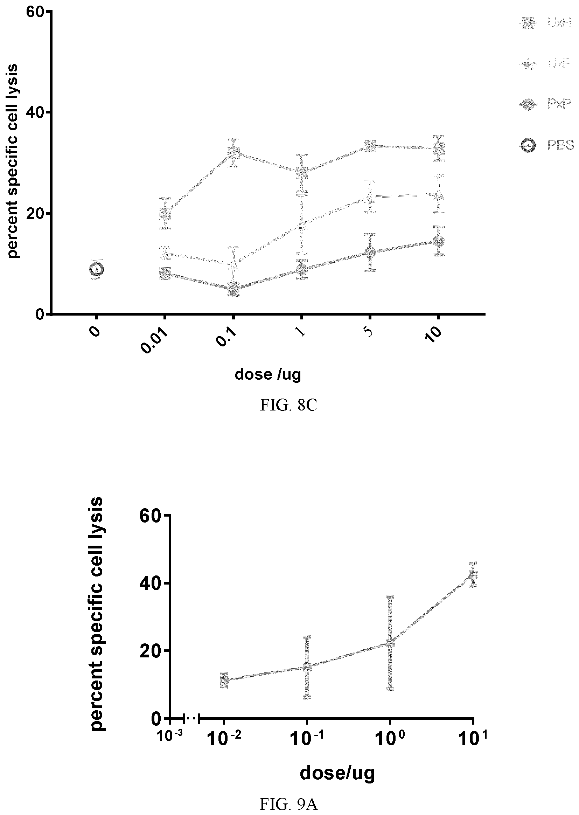

These experimental data fully demonstrate that the multicellular targeting liposome of the present disclosure can effectively mediate the effector cells to effectively kill the target cells, and the effector cells may include unstimulated lymphocytes, in-vitro amplified lymphocytes, in-vitro activated lymphocytes, in-vitro differentiated lymphocytes. The cytotoxic effect is dependent on the membrane lysis effect of cell lysis, and is dose-dependent. In addition, the multi-targeting antibodies can effectively mediate the killing of effector cells against different target cells expressing the same antigen (FIG 9A CD19 + SUP-B15 cells).

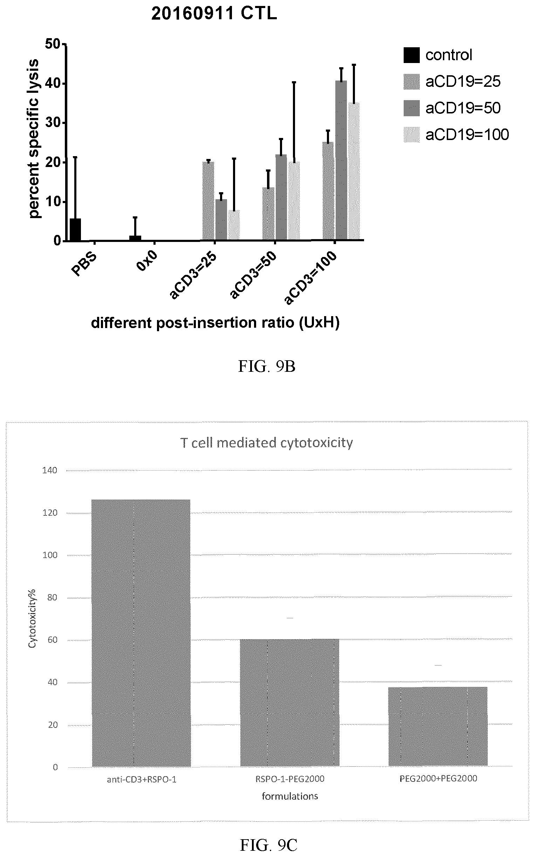

The results of replacing different antibody molecules or target cells are shown in FIGs. 9A, 9B, and 9C. The predominant antibody of FIG. 9A is replaced with anti-CD3 antibodies of different clones (2C11) and different antibody forms (IgG). FIG. 9B is a multicellular targeting liposome with the same predominant antibody and auxiliary antibody, while the target cells are replaced with CD19 + Human leukemia SUP-B15 cells. The auxiliary antibody of FIG. 9C is replaced with a different targeting molecule (RSPO-1 protein) that targets a different antigen (LGR), and the target cells are replaced with non-small cell lung cancer A549 cells (solid tumor model).

These experimental data fully demonstrate that the first antibody and auxiliary antibody of the multicellular targeting liposome of the present disclosure may be a targeting molecule of any form and structure that targets any antigen, and the target cell and effector cell may be any cell that the predominant antibody and auxiliary antibody can bind to. The multicellular targeting liposome mediates the interaction between immune effector cells and target cells based on the targeting ability. According to the types of different diseases, we may effectively mediate the effect of effector cells on target cells by replacing any targeting molecules (predominant antibody and auxiliary antibody).

### Embodiment 5 Evaluation of the activation effect of multicellular targeting liposome stimulating effector cells

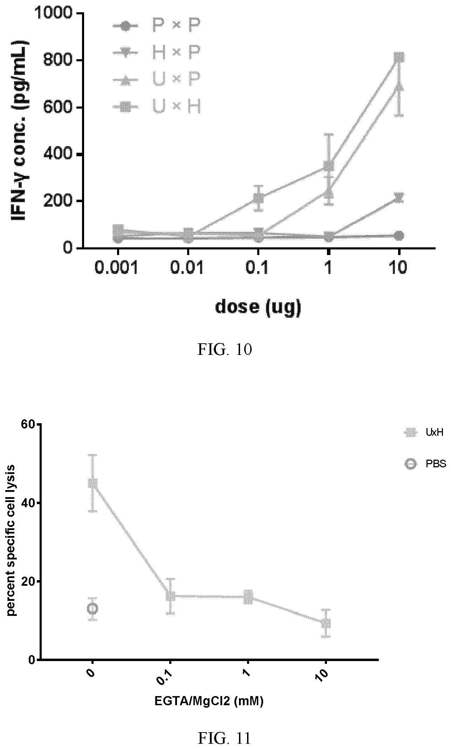

Effector cells (unstimulated human lymphocytes) and target cells (Raji cells) are resuspended in phenol red-free complete medium, and the cell concentrations are 1^{∗}10^6 cells/ml and 1.11^{∗}10^5 cells/ml, respectively. Adding 100 µl effector cells and 90 µl target cells to a 96-well plate and mixing by pipetting to plate the cells. At this time, the ratio of target cells to effector cells is 10: 1. Adding 10 µl of targeting liposomes of different concentrations to each well, and mixing by pipetting. Placing the 96-well plate in a cell incubator, incubating for 24 h. After the incubation, centrifuging the 96-well plate at 250 g for 4 min. After centrifugation, pipetting 50 µl of the supernatant to a new 96-well plate. Detecting the IFN-γ in the supernatant by ELISA method. The result shown in FIG. 10 fully demonstrates that the multicellular targeting liposome of the present disclosure can effectively mediate the unstimulated lymphocytes, in-vitro amplified lymphocytes, in-vitro activated lymphocytes, in-vitro differentiated lymphocytest to effectively kill the target cells, while promoting the activation of effector cells, the production and release of cytokines. The produced cytokines have anti-tumor activity and is dose-dependent.

### Embodiment 6 Study on the mechanism of multicellular targeting liposome

Effector cells (unstimulated human lymphocytes) and target cells (Raji cells) are resuspended in phenol red-free complete medium, and the cell concentrations are 1.11^{∗}10^6 cells/ml and 1.11^{∗}10^5 cells/ml, respectively. Adding 90 µl effector cells and 90 µl target cells to a 96-well plate and mixing by pipetting to plate the cells. At this time, the ratio of target cells to effector cells is 10: 1. Adding 10 µl of multicellular targeting liposome to each well, and mixing by pipetting. Adding 10 µl EGTA/MgCl₂ to each well with the final concentration of OmM, 0.1mM, 1mM and 10mM, and mixing by pipetting. Placing the 96-well plate in a cell incubator, incubating for 24 h. 45 min before the end of the incubation, adding 10 µL lysate to the volume-correcting control well and the targeting cell maximum lysis well. After the incubation, centrifuging the 96-well plate at 250 g for 4 min. After centrifugation, pipetting 50 µl of the supernatant to a new 96-well plate, adding 50 µl of LDH substrate to the new well plate, and incubating at 22 °C for 15 min. After incubation, detecting the absorbance in a microplate reader at a wavelength of 490 nm.

The result shown in FIG. 11 fully demonstrates that the killing effect of the effector cells mediated by the multicellular targeting liposome on target cells is based on the calcium ion-dependent granzyme/perforin pathway of the effector cells.

### Embodiment 7 Evaluation of targeting drug delivery effect of multicellular targeting liposome

The drug-loading multicellular targeting liposomes are prepared by the post-insertion method. For multicellular targeting drug-loading liposome, the prepared anti-CD19 and anti-CD3 Fab' fragment-polymer-lipid linker (DSPE-PEG2000-Mal- Fab') is mixed with the dasatinib drug-loading liposome in a molar ratio (0.1-5): (0.1-5): 1000, and the reaction is performed at 37 ° C under the protection of N₂ with shaking for 24 h. Then, the liposomes are washed 5 times by 300KD ultrafiltration to remove the unlinked antibody Fab' fragments to obtain the multicellular targeting dasatinib liposome.

Effector cells (unstimulated lymphocytes) and target cells (Raji cells) are resuspended in phenol red-free complete medium, and the cell concentrations are 1^{∗}10^6 cells/ml and 1.11^{∗}10^5 cells/ ml, respectively. Adding 100 µl effector cells and 90 µl target cells to a 96-well plate and mixing by pipetting to plate the cells. At this time, the ratio of target cells to effector cells is 1:10. Adding 10 µl of multicellular targeting drug-loading liposomes with different dasatinib concentrations and same lipid concentrations to each well, and mixing by pipetting. Placing the 96-well plate in a cell incubator, incubating for 24 h. 45 min before the end of the incubation, adding 10 µl lysate to the volume-correcting control well and the targeting cell maximum lysis well. After the incubation, centrifuging the 96-well plate at 250 g for 4 min. After centrifugation, pipetting 50 µl of the supernatant to a new 96-well plate, adding 50µl of LDH substrate to the new well plate, and incubating at 22 °C for 15 min. After incubation, detecting the absorbance in a microplate reader at a wavelength of 490 nm.

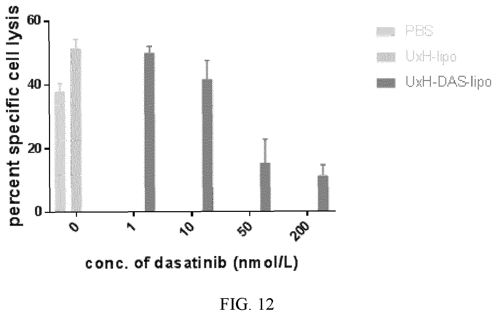

The result shown in FIG. 12 fully demonstrates that by linking the predominant antibody and the auxiliary antibody to the drug-loading liposome, the drug-loading effect can be fully exerted on the basis of the multicellular targeting mechanism.

The above are only some preferred embodiments of the present disclosure instead of limitations on the present disclosure in any form or substance. It should be noted that, for those skilled in the art, improvements and supplements may be made without departing from the method of the present disclosure, the improvements and supplements shall also be covered by the protection of the present disclosure. The equivalent changes of alternations, modifications and evolutions can be made by those skilled in the art using the technical contents revealed above and without departing from the spirit and scope of the present disclosure, and those equivalent changes are regarded as equivalent embodiments of the present disclosure. Meanwhile, any alterations, modifications, and evolutions of any equivalent changes made to the above embodiments according to the essential technology of the present disclosure still fall within the scope of the technical solution of the present disclosure.

## Claims

1. A multicellular targeting liposome, comprising a liposome, and a predominant antibody and an auxiliary antibody modified on a surface of the liposome, wherein the predominant antibody specifically binds to a target molecule on a surface of a target cell, and the auxiliary antibody specifically binds to an immune effector cell.

2. The multicellular targeting liposome according to claim 1, wherein the multicellular targeting liposome is capable of simultaneously or sequentially binding to the immune effector cell and the target cell, such that the immune effector cell recognizes the target cell and activates the immune effect.

3. The multicellular targeting liposome according to claim 1, wherein the molar ratio of the predominant antibody to the auxiliary antibody on each liposome is between 100/1 and 1/1.

4. The multicellular targeting liposome according to claim 1, wherein each liposome has 1-1000 predominant antibodies and 1-1000 auxiliary antibodies on the surface.

5. The multicellular targeting liposome according to claim 1, wherein the predominant antibody and the auxiliary antibody are displayed on the surface of the liposome through covalent linkage or hydrophobic, hydrophilic interaction, respectively.

6. The multicellular targeting liposome according to claim 1, wherein an antigen targeted by the predominant antibody is selected from a group consisting of a microbial antigen, a tumor-related antigen, a tumor cell surface specific antigen, a highly expressed antigen on a tumor cell surface, and a highly expressed antigen in a tumor tissue or a tumor blood vessel; an antigen targeted by the auxiliary antibody is an antigen expressed by a tumor cell, a lymphocyte or a myeloid cell.

7. The multicellular targeting liposome according to claim 1, which is capable of simultaneously or sequentially binding to a tumor cell, a lymphocyte, or a myeloid cell.

8. The multicellular targeting liposome according to claim 1, wherein the multicellular targeting liposome targets and binds to a T lymphocyte, an NK cell, an NKT cell, a macrophage, or a neutrophil.

9. The multicellular targeting liposome according to claim 1, wherein the multicellular targeting liposome targets and binds to an unactivated T cell, or an in-vitro activated or amplified T cell.

10. The multicellular targeting liposome according to claim 9, wherein the T lymphocyte is selected from a group consisting of an antigen-specific T lymphocyte, a tumor infiltrating lymphocyte, a cytotoxic T cell, a helper T cell, a lymphokine-activated killer cell, a γδ T cell, a chimeric antigen receptor T cell, and a T cell receptor chimeric T cell.

11. The multicellular targeting liposome according to claim 1, wherein a form of the predominant antibody and the auxiliary antibody is selected from a group consisting of IgG, Fab' fragment, F(ab')2 fragment, Fab fragment, single-chain Fv fragment, minibody, nanobody, unibody, and diabody.

12. The multicellular targeting liposome according to claim 1, wherein an antigen targeted by the primary antibody is selected from a group consisting of CD19, CD20, PSMA, Her2/neu, EGFR and LGR5.

13. The multicellular targeting liposome according to claim 1, wherein the auxiliary antibody is selected from a group consisting of CD3 antibody and a fragment, a PD1 antibody and a fragment, a CTLA4 antibody and a fragment, a CD40L antibody and a fragment, or a combination thereof.

14. The multicellular targeting liposome according to claim 1, wherein components of the liposome comprise phosphatidylcholine, cholesterol, a lipid linked to the predominant antibody, and a lipid molecule linked to the auxiliary antibody.

15. The multicellular targeting liposome according to claim 1, wherein the liposome is a blank liposome or a drug-loading liposome.

16. A method for preparing the multicellular targeting liposome according to any one of claims 1 to 15, comprising: (1) connecting the predominant antibody and the auxiliary antibody with a lipid molecule respectively to obtain a predominant antibody-lipid molecule and an auxiliary antibody-lipid molecule; (2) mixing the predominant antibody-lipid molecule and the auxiliary antibody-lipid molecule with a constructed liposome, and incubating to obtain a mixed solution; (3) dialyzing or ultrafiltrating the mixed solution to remove unloaded antibody and antibody-lipid complexes, and obtaining the multicellular targeting liposome.

17. Use of the multicellular targeting liposome of any one of claims 1 to 15 in preparing immunotherapeutic drugs and antitumor drugs.

18. A method for treating tumor, comprising: administering the multicellular targeting liposome of any one of claims 1 to 15 to a tumor patient.
